# EUROPÄISCHE PATENTANMELDUNG

(11) **EP 3 552 658 A1**
(43) Veröffentlichungstag der Anmeldung: **16.10.2019**
(21) Anmeldenummer: 18211385.2
(22) Anmeldetag: 10.12.2018
(51) Int. Cl.: A61M 37/00

(54) **ANTRIEBSMODUL UND NADELMODUL FÜR EIN HANDGERÄT ZUM WIEDERHOLTEN LOKALEN AUFSTECHEN EINER MENSCHLICHEN ODER EINER TIERISCHEN HAUT SOWIE HANDGERÄT**

(71) Anmelder: MT.DERM GmbH, 14167 Berlin (DE)
(72) Erfinder: Kelp, Martin, 12205 Berlin (DE); Scherkowski, Dirk, 12489 Berlin (DE); Pohl, David, 12051 Berlin (DE)
(74) Vertreter: Bittner, Thomas L.

(57) **Zusammenfassung**

Die Erfindung betrifft ein Antriebsmodul (2) für ein Handgerät zum wiederholten lokalen Aufstechen einer menschlichen oder einer tierischen Haut mit einem Antriebsmodul-Gehäuse (1a); einer maschinellen Antriebseinrichtung (4), die in dem Antriebsmodul-Gehäuse (1a) angeordnet und eingerichtet ist, eine Antriebskraft bereitzustellen; und einem antriebsseitigen Kopplungselement, welches in dem Antriebsmodul-Gehäuse (1a) angeordnet ist, an die Antriebseinrichtung (4) koppelt und eingerichtet ist, die bereitgestellte Antriebskraft auf ein abtriebsseitiges Kopplungselement, welches außerhalb des Antriebsmodul-Gehäuses (1a) an einem Nadelmodul (3) und dort an eine Stecheinrichtung (7) koppelnd angeordnet ist, kontaktlos zu übertragen. Ferner werden ein Nadelmodul (3) für ein Handgerät zum wiederholten lokalen Aufstechen einer menschlichen oder einer tierischen Haut sowie ein Handgerät zum wiederholten lokalen Aufstechen einer menschlichen oder einer tierischen Haut bereitgestellt.

## Beschreibung

Die Erfindung betrifft ein Antriebsmodul und ein Nadelmodul für ein Handgerät zum wiederholten lokalen Aufstechen einer menschlichen oder einer tierischen Haut sowie ein Handgerät.

### Hintergrund

Derartige Handgeräte werden genutzt, um eine Haut wiederholt lokal aufzustechen. Das Aufstechen der Haut kann dazu dienen, ein Tattoo oder permanentes Make-up herzustellen, in dem ein Farbstoff in die Haut eingebracht wird. Auch kosmetische oder medizinische Anwendungen können vorgesehen sein, um mittels lokalem Aufstechen der Haut Wirkstoffe einzubringen.

Solche Handgeräte weisen regelmäßig ein Antriebsmodul und ein hieran gekoppeltes Nadelmodul auf. Das Antriebsmodul umfasst eine maschinelle Antriebseinrichtung, mit der eine maschinell erzeugte Antriebskraft wiederholt oder zyklisch bereitgestellt wird. Über eine Kopplungseinrichtung wird die Antriebskraft auf eine Stecheinrichtung in dem an das Antriebsmodul koppelnden Nadelmodul übertragen, um eine Stechnadel der Stecheinrichtung wiederholt oder zyklisch vor- und zurückzubewegen.

### Zusammenfassung

Aufgabe der Erfindung ist es, ein Antriebsmodul sowie ein Nadelmodul für ein Handgerät zum wiederholten lokalen Aufstechen einer menschlichen oder einer tierischen Haut sowie ein Handgerät anzugeben, bei denen die im Antriebsmodul maschinell erzeugte Antriebskraft auf verbesserte Art und Weise auf die Stecheinrichtung einkoppelbar ist.

Zur Lösung sind ein Antriebsmodul sowie ein Nadelmodul für ein Handgerät zum wiederholten lokalen Aufstechen einer menschlichen oder einer tierischen Haut nach den unabhängigen Ansprüchen 1 und 7 geschaffen. Weiterhin ist ein Handgerät nach dem nebengeordneten Anspruch 12 vorgesehen. Ausgestaltungen sind Gegenstand von abhängigen Unteransprüchen.

Nach einem Aspekt ist ein Antriebsmodul für ein Handgerät zum wiederholten lokalen Aufstechen einer menschlichen oder einer tierischen Haut geschaffen, welches Folgendes aufweist: ein Antriebsmodul-Gehäuse; eine maschinelle Antriebseinrichtung, die in dem Antriebsmodul-Gehäuse angeordnet und eingerichtet ist, eine Antriebskraft bereitzustellen; und ein antriebsseitiges Kopplungselement, welches in dem Antriebsmodul-Gehäuse angeordnet ist, an die Antriebseinrichtung koppelt und eingerichtet ist, die bereitgestellte Antriebskraft auf ein abtriebsseitiges Kopplungselement, welches außerhalb des Antriebsmodul-Gehäuses an einem Nadelmodul und dort an eine Stecheinrichtung koppelnd angeordnet ist, kontaktlos zu übertragen.

Nach einem weiteren Aspekt ist ein Nadelmodul für ein Handgerät zum wiederholten lokalen Aufstechen einer menschlichen oder einer tierischen Haut geschaffen, welches Folgendes aufweist: ein Nadelmodul-Gehäuse; eine Stecheinrichtung, die in dem Nadelmodul-Gehäuse angeordnet ist, derart, dass ein Stechwerkzeug der Stecheinrichtung durch eine vorderseitigen Gehäuseöffnung vor und zurück bewegt werden kann; und ein abtriebsseitiges Kopplungselement, welches in dem Nadelmodul-Gehäuse angeordnet ist, an die Stecheinrichtung koppelt und eingerichtet ist, zum Vor- und Zurückbewegen des Stechwerkzeugs eine maschinell erzeugte Antriebskraft mittels kontaktloser Kraftübertragung von einem antriebsseitigen Kopplungselement zu empfangen, das in einem Antriebsmodul und dort an eine maschinelle Antriebseinrichtung koppelnd angeordnet ist.

Weiterhin ist ein Handgerät zum wiederholten lokalen Aufstechen einer menschlichen oder einer tierischen Haut mit dem Antriebsmodul und dem hieran lösbar gekoppelten Nadelmodul geschaffen.

Das Antriebsmodul kann als wiederverwendbares Modul zur Mehrfachnutzung ausgeführt sein. Die maschinelle Antriebseinrichtung in dem Antriebsmodul-Gehäuse kann mit einem elektrischen Motor gebildet sein.

Die Antriebseinrichtung kann eingerichtet sein, die Antriebskraft mit einer Wiederholfrequenz von 20 bis 180 Hz bereitzustellen. Alternativ kann eine manuell betätigbare Antriebseinrichtung vorgesehen sein.

Mithilfe des vorgeschlagenen Antriebsmoduls und des vorgeschlagenen Nadelmoduls ist es ermöglicht, die im Antriebsmodul maschinell erzeugte Antriebskraft kontaktlos auf die Stecheinrichtung im Nadelmodul zu übertragen. Hierdurch sind eine mechanische Kopplung zwischen den Kopplungselementen im Antriebsmodul und im Nadelmodul und die hiermit regelmäßig verbundenen Probleme vermieden. Beispielsweise ist es nicht erforderlich, eine kontaktbehaftete mechanische Kopplung zwischen dem Kopplungselement im Antriebsmodul und dem Kopplungselement in dem Nadelmodul bereitzustellen. Dieses ermöglicht eine weniger aufwendige und weniger komplizierte Herstellung des jeweiligen Gehäuses für das Antriebsmodul und das Nadelmodul.

Das Stechwerkzeug der Stecheinrichtung kann als Einzelnadel oder als Gruppe von Nadeln zum lokalen Aufstechen der Haut ausgebildet sein.

Das antriebsseitige Kopplungselement kann eingerichtet sein, die bereitgestellte Antriebskraft durch eine Wandung des Antriebsmodul-Gehäuses hindurch kontaktlos zu übertragen. Die Wandung im Bereich der kontaktlosen Kraftübertragung kann als vollständig geschlossene Wandung des Antriebsmodul-Gehäuses ausgebildet sein.

Das antriebsseitige Kopplungselement kann zum kontaktlosen Übertragen der Antriebskraft bewegbar in dem Antriebsmodul-Gehäuse gelagert sein. Das antriebsseitige Kopplungselement kann zum kontaktlosen Übertragen der Antriebskraft wenigstens eine der folgenden Bewegungen ausführen: Drehbewegung und Linearbewegung. Die antriebsseitige Bewegung des Kopplungselements bewirkt in Folge der kontaktlosen Kraftübertragung auf das abtriebsseitige Kopplungselement eine zyklische oder wiederholte Bewegung eines Stechwerkzeugs der Stecheinrichtung.

Das Antriebsmodul-Gehäuse kann, zumindest in einem vorderen Gehäuseabschnitt, in welchem das Antriebsmodul-Gehäuse an das Nadelmodul koppelbar ist, ein geschlossenes Gehäuse sein. Das Antriebsmodul-Gehäuse kann als geschlossenes Gehäuse gegen die Umgebung abgedichtet sein, zum Beispiel fluiddicht, insbesondere flüssigkeitsdicht. Hierdurch ist vermieden, dass die bei der Benutzung des Handgeräts zum wiederholten lokalen Aufstechen der Haut gegebenenfalls im Aufstechbereich austretende Körperflüssigkeit ins Innere des Antriebsmodul-Gehäuses gelangt. Hierbei können wahlweise gedichtete Kabeldurchführungen an dem Antriebsmodul-Gehäuse vorgesehen sein, um zum Beispiel Kabel für eine Spannungsversorgung der maschinellen Antriebseinrichtung durch die Wandung des Antriebsmodul-Gehäuses hindurchzuführen. Das gesamte Gehäuse des Antriebsmodul-Gehäuses kann hierbei als geschlossenes und wahlweise gegen die Umgebung abgedichtetes Gehäuse ausgeführt sein.

Das antriebsseitige Kopplungselement kann ein oder mehrere magnetische Bauteile aufweisen, welche eingerichtet sind, zum kontaktlosen Übertragen der Antriebskraft ein Magnetfeld bereitzustellen. Das oder die magnetischen Bauteile des antriebsseitigen Kopplungselements können einem magnetischen Gegenbauteil am abtriebsseitigen Kopplungselement zugeordnet sein, um so zwischen den magnetischen Bauteilen die maschinell erzeugte Antriebskraft kontaktlos zu übertragen. Die magnetischen Bauteile können einen oder mehrere Permanentmagnete aufweisen. Entlang einer Bewegungsrichtung des Stechwerkzeugs beim Ein- und Ausfahren und / oder quer hierzu können einander zugeordnete magnetische Bauteile vorgesehen sein. Bei dieser oder anderen Ausführungsformen kann eine von der maschinellen Antriebseinrichtung bereitgestellte Drehbewegung in die Linearbewegung der Stechnadel der Stecheinrichtung umgesetzt werden.

Das antriebsseitige Kopplungselement kann einer antriebsmodulseitigen Aufnahme, in welcher das abtriebsseitige Kopplungselement bei angekoppeltem Nadelmodul wenigstens teilweise angeordnet ist, mehrseitig gegenüberliegend ausgebildet sein. Die antriebsmodulseitige Aufnahme für das Nadelmodul kann beispielsweise als eine Einsteck- oder eine Schraubaufnahme ausgeführt sein. Auch zum Ausbilden einer Klickverbindung zwischen Antriebsmodul und Nadelmodul kann die Aufnahme hergerichtet sein.

Das Nadelmodul kann ein Einwegmodul sein. Auf diese Weise wird ein hohen hygienischen Standards genügender Einsatz des Handgeräts unterstützt.

In dem Nadelmodul-Gehäuse kann eine Rückstelleinrichtung angeordnet sein, welche an die Stecheinrichtung koppelt und eingerichtet ist, ein Zurückverlagern der Stechnadel zwischen einer Einstechstellung und einer Ausgangstellung wenigstens zu unterstützen. Zumindest in der Einstechstellung ist eine Nadelspitze des Stechwerkzeugs durch eine vorderseitige Gehäuseöffnung an dem Nadelmodul ausgefahren, derart, dass mittels der Nadelspitze die Haut aufgestochen werden kann. Nachdem die Stecheinrichtung mit dem Stechwerkzeug aufgrund der Antriebskraft in die Ausgangsstellung oder die Einstechstellung verlagert wurde, unterstützt die Rückstelleinrichtung das Zurückbewegen in die andere Stellung. Die Rückverlagerung kann ausschließlich durch die Rückstelleinrichtung bewirkt werden. Alternativ kann vorgesehen sein, dass die Rückstelleinrichtung eine Rückholkraft unterstützt, die von der Wechselwirkung des antriebsseitigen und des abtriebsseitigen Kopplungselements bereitgestellt ist. Die Rückstelleinrichtung kann beispielsweise einen Federmechanismus umfassen, mit dem das Stechwerkzeug oder die Stecheinrichtung gegen eine Verlagerung in eine der Stellungen vorgespannt ist, beispielsweise eine Spiralfeder.

Das abtriebsseitige Kopplungselement kann ein magnetisches Bauteil aufweisen, welches eingerichtet ist, an ein Magnetfeld des antriebsseitigen Kopplungselementes zum kontaktlosen Übertragen der Antriebskraft zu koppeln. In Verbindung mit der Nutzung eines oder mehrerer magnetischer Bauteile bei dem abtriebsseitigen Kopplungselement gelten die vorangehend in Verbindung mit dem antriebsseitigen Kopplungselement gemachten Erläuterungen entsprechend.

Die vorangehend im Zusammenhang mit dem Antriebsmodul und / oder dem Nadelmodul erläuterten Ausgestaltungen können bei dem Handgerät zum wiederholten lokalen Aufstechen einer menschlichen oder einer tierischen Haut entsprechend vorgesehen sein.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine schematische Darstellung eines Handgeräts zum wiederholten lokalen Aufstechen einer menschlichen oder einer tierischen Haut mit einem Antriebsmodul und einem hieran lösbar koppelnden Nadelmodul;
- Fig. 2: eine schematische Darstellung einer Kopplungseinrichtung zum kontaktlosen Übertragen einer Antriebskraft, bei der eine Drehbewegung eines antriebsseitigen Kopplungselements in eine Linearbewegung eines abtriebsseitigen Kopplungselements gewandelt wird;
- Fig. 3: eine schematische Darstellung einer Kopplungseinrichtung zum kontaktlosen Übertragen einer Antriebskraft, bei der eine Linearbewegung des antriebsseitigen Kopplungselements in eine Linearbewegung des abtriebsseitigen Kopplungselements gewandelt wird;
- Fig. 4: eine schematische Darstellung einer Kopplungseinrichtung zum kontaktlosen Übertragen einer Antriebskraft, bei der die Linearbewegung des antriebsseitigen Kopplungselements in eine Linearbewegung des abtriebsseitigen Kopplungselements gewandelt wird;
- Fig. 5: eine schematische Darstellung einer Kopplungseinrichtung zum kontaktlosen Übertragen einer Antriebskraft, bei der das abtriebsseitige Kopplungselement zwischen Bauteilen des antriebsseitigen Kopplungselements angeordnet ist; und
- Fig. 6: eine schematische Darstellung einer Kopplungseinrichtung zum kontaktlosen Übertragen einer Antriebskraft, bei der das abtriebsseitige Kopplungselement zwischen Bauteilen des antriebsseitigen Kopplungselements angeordnet ist.

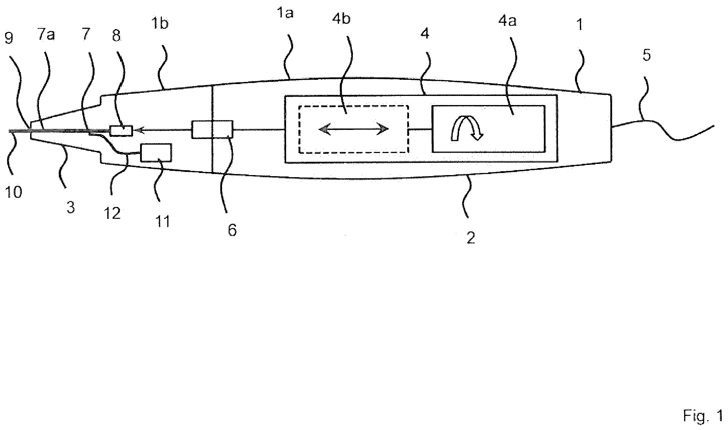

Fig. 1 zeigt eine schematische perspektivische Darstellung eines Handgeräts 1 zum wiederholten lokalen Aufstechen einer menschlichen oder tierischen Haut mit einem Gehäuse 1. Das Gehäuse 1 ist modulartig aufgebaut mit einem Antriebsmodul-Gehäuse 1a eines Antriebsmoduls 2 und einem Nadelmodul-Gehäuse 1b eines Nadelmoduls 3, welches lösbar an das Antriebsmodul 2 koppelt.

In dem Antriebsmodul-Gehäuse 1a ist eine Antriebseinrichtung 4 vorgesehen, die in der dargestellten Ausführungsform einen Motor 4a sowie einen optionalen Wandlungsmechanismus 4b aufweist. Mittels des Motors 4a wird bei dem Beispiel eine drehende Antriebsbewegung (drehende Antriebskraft) bereitgestellt, die mit Hilfe des Wandlungsmechanismus 4b in eine lineare Antriebskraft in Längsrichtung des Gehäuses 1 gewandelt wird, sei es zum Ausführen eines Einzelhubs oder für eine repetitive Bewegung. Derartige Wandlungsmechanismen sind als solche in verschiedenen Ausführungsformen bekannt, weshalb dies hier keiner weiteren Erläuterung bedarf. Zum Beispiel kann ein Mechanismus mit einer Taumelscheibe und einem Pleuel vorgesehen sein. Aber auch andere Wandlungsmechanismen sind als solche bekannt. Alternativ kann die drehende Antriebsbewegung auf das Nadelmodul 3 übertragen werden.

Zur Energieversorgung ist das Handgerät über eine Zuleitung 5 an eine Versorgungsspannung angeschlossen. Die Zuleitung 5 kann ergänzend Kabel umfassen, über die Steuersignale zwischen einem Steuergerät (nicht dargestellt) und dem Handgerät übertragen werden.

Die von der Antriebseinrichtung 4a maschinell erzeugte Antriebskraft wird über eine Kopplungseinrichtung 6 auf eine Stecheinrichtung 7 mit einem Stechwerkzeug 7a gegeben, welches in einer Werkzeugaufnahme 8 angeordnet ist. Im Fall der Verwendung einer oder mehrerer Nadeln als Stechwerkzeug 7a kann es sich bei der Werkzeugaufnahme 8 um einen Nadelschaft handeln, der in dem Nadelmodul-Gehäuse 1b in einer zugeordneten Führung angeordnet ist, so dass die repetitive oder zyklische lineare Antriebskraft für eine Vor- und Rückwärtsbewegung von Werkzeugaufnahme 8 mit Stechwerkzeug 7a umgesetzt werden kann. Das Stechwerkzeug 7a ist durch eine Gehäuseöffnung 9 frontseitig an dem Nadelmoduf-Gehäuse 1b geführt, so dass zumindest in einer ausgefahrenen Stellung eine Stechwerkzeugspitze 10 außerhalb des Nadelmodul-Gehäuses 1b angeordnet ist, um die zu bearbeitende Haut aufzustechen. Die Gehäuseöffnung 9 ist bei der gezeigten Ausführungsform am vorderen Ende des Nadelmodul-Gehäuses 1b an dem Nadelmodul 3 gebildet, welches lösbar an dem Antriebsmodul-Gehäuse 1a angeordnet ist.

Bei der gezeigten Ausführungsform des Handgeräts ist in dem Gehäuse 1 weiterhin ein Reservoir 11 vorgesehen, welches zur Aufnahme einer in die Haut einzubringenden Substanz dient, zum Beispiel einer Farbe im Fall des Herstellens eines Tattoos oder von permanentem Makeup. Über einen Ausfluss 12 wird die Substanz an das Stechwerkzeug 7a herangeführt, um so an der Stechwerkzeugspitze 10 die Substanz zum Einbringen in die Haut bereitzustellen. Alternativ kann das Reservoir 11 auch im Antriebsmodul-Gehäuse 1a oder außerhalb des Gehäuses 1 angeordnet sein. Verschiedene Ausgestaltungen für ein solches Reservoir und das Bereitstellen der einzubringenden Substanz im Bereich des Stechwerkzeugs 7 sind als solche bekannt, beispielsweise auch unter Verwendung einer Pumpeinrichtung an dem Reservoir 13.

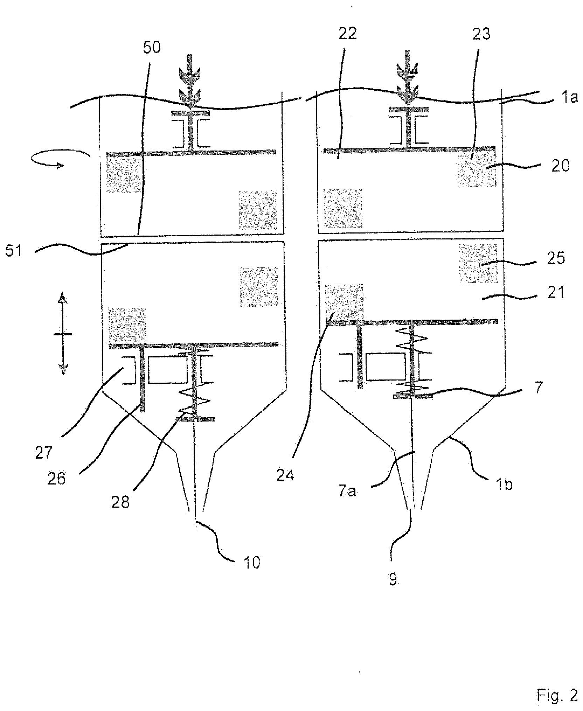

Im Folgenden wird auf die Fig. 2 bis 6 Bezug genommen, die verschiedene Beispiele für die Kopplungseinrichtung 6 zeigen, mit der die in dem Antriebsmodul 2 mittels der Antriebseinrichtung 4, insbesondere mit dem Motor 4a, maschinell erzeugte Antriebskraft auf die Stecheinrichtung 7 in dem Nadelmodul 3 kontaktlos übertragen wird, insbesondere durch einander gegenüberliegende geschlossene Wandungen des Antriebsmodul-Gehäuses 1a und optional auch des Nadelmodul-Gehäuses 1b. Für die gleichen Merkmale werden in den Fig. 2 bis 6 dieselben Bezugszeichen verwendet.

Fig. 2 zeigt eine schematische Darstellung der Kopplungseinrichtung 6 mit einem antriebsseitigen Kopplungselement 20 in dem Antriebsmodul-Gehäuse 1a des Antriebsmoduls 2 und einem hieran funktionell koppelnden abtriebsseitigen Kopplungselements 21 in dem Nadelmodul-Gehäuse 1b des Nadelmoduls 3. Bei der gezeigten Ausführung ist das antriebsseitige Kopplungselement 20 drehbar gelagert und weist magnetische Bauteile 22, 23 auf. Im Betrieb wird das antriebsseitige Kopplungselement 20 mithilfe des Motors 4 gedreht. Magnetische Gegenbauteile 24, 25 des abtriebsseitigen Kopplungselements 21 werden hierdurch abwechselnd abgestoßen (vgl. linke Darstellung in Fig. 2) und angezogen (vgl. rechte Darstellung in Fig. 2). Die Drehbewegung des antriebsseitigen Kopplungselements 20 wird so in eine Linearbewegung des abtriebsseitigen Kopplungselements 21 umgesetzt. Das abtriebsseitige Kopplungselement 21 koppelt an die Stecheinrichtung 7 mit dem Stechwerkzeug 7a. Die Linearbewegung des abtriebsseitigen Kopplungselements 21 in Längsrichtung führt somit dazu, dass die Stechwerkzeugspitze 10 durch die vorderseitige Gehäuseöffnung 9 zyklisch oder wiederholt aus- und eingefahren wird, was mit der Wiederholfrequenz der im Antriebsmodul 2 bereitgestellten Antriebskraft erfolgt.

Bei dem gezeigten Beispiel in Fig. 2 ist ein Führungselement 26 an dem abtriebsseitigen Kopplungselement 21 vorgesehen, welches in einer Führung 27 geführt ist, wenn die Linearbewegung vor und zurück ausgeführt wird.

Mittels eines Federmechanismus 28 wird für das Stechwerkzeug 7a eine Rückstellkraft bereitgestellt.

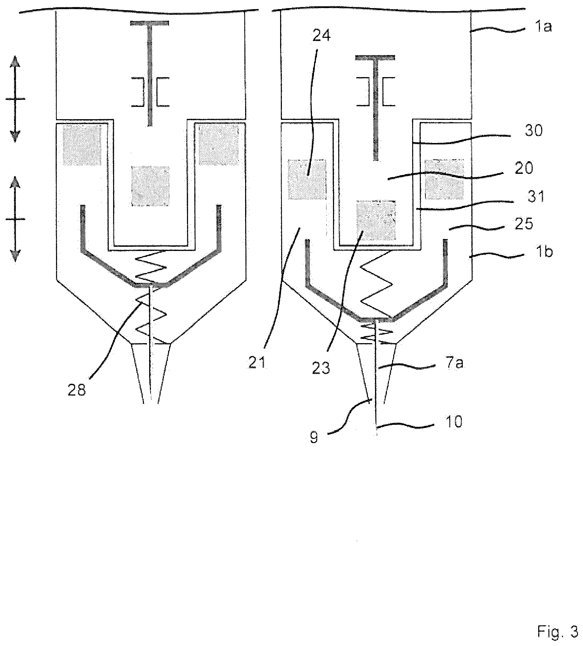

Bei dem in Fig. 3 gezeigten Beispiel ist ein vorderseitiger Gehäuseabschnitt 30 des Antriebsmoduls 2 in einer zugeordneten nadelmodulseitigen Aufnahme 31 angeordnet, derart, dass das magnetische Bauteil 23 des antriebsseitigen Kopplungselements 20, welches in dem vorstehenden Gehäuseabschnitt 30 angeordnet ist, zwischen den magnetischen Gegenbauteilen 24, 25 des abtriebsseitigen Kopplungselements 21 lagert. Eine Linearbewegung des antriebsseitigen Kopplungselements 20 führt dann zur Linearbewegung des abtriebsseitigen Kopplungselements 21, sodass die Stechwerkzeugspitze 10 aus- und eingefahren wird.

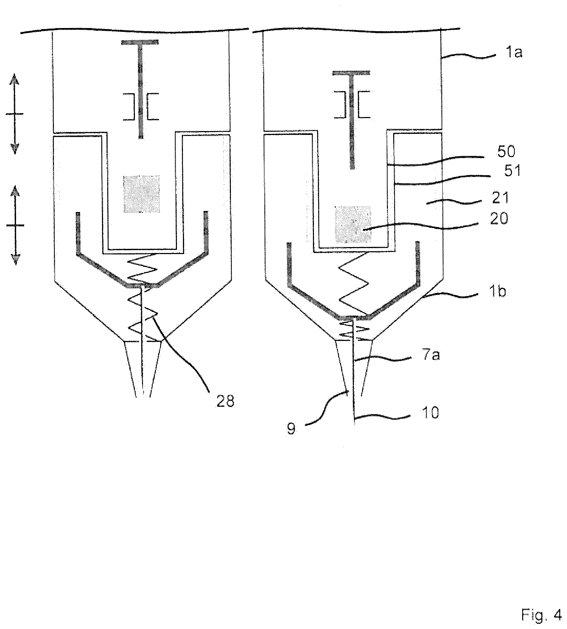

Fig. 4 zeigt ein Beispiel vergleichbar der Ausführung in Fig. 3, wobei dem antriebsseitigen Kopplungselement 20 abtriebsseitig zugeordnete und im gezeigten Beispiel magnetflussführende Teile des abtriebsseitigen Kopplungselements 21 aus weichmagnetischem Material bestehen. Durch deren Materialeigenschaft, Gestaltung und / oder Abstand zum antriebsseitigen Kopplungselement 20 kann ein Übertragungsverhalten zwischen dem Antriebsmodul 4 und dem Stechwerkzeug 7a konstruktiv und durch geeignete Zusatzeinrichtungen (nicht dargestellt) beeinflusst und bedarfsgerecht eingestellt werden.

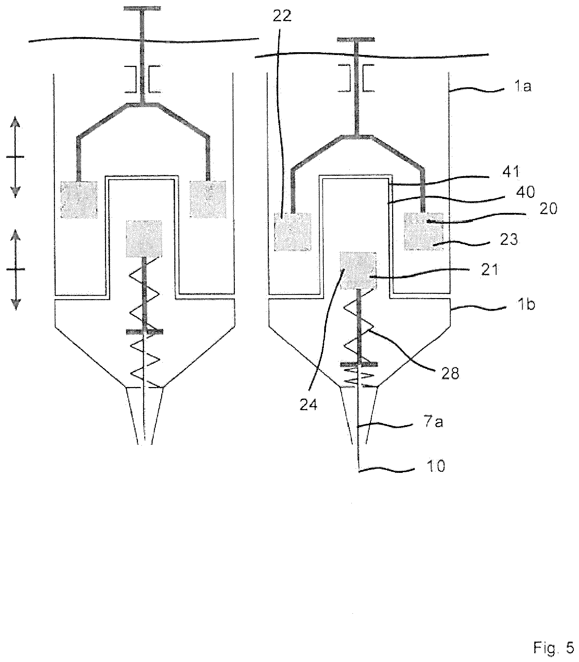

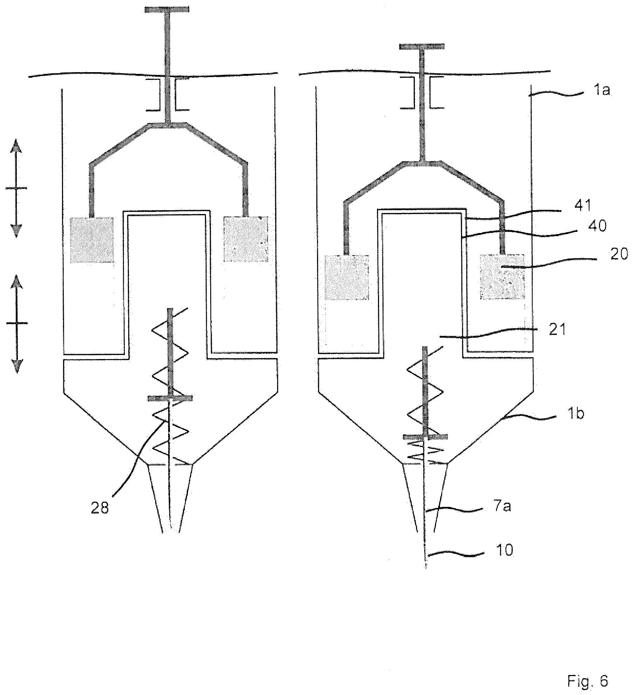

Bei den Beispielen in den Fig. 5 und 6 ist ein rückseitiger vorstehender Gehäuseabschnitt 40 des Nadelmoduls 3 in einer zugeordneten antriebsmodulseitigen Aufnahme 41 angeordnet. Auf diese Weise ist das magnetische Gegenbauteil 24, welches in dem rückseitig vorstehenden Gehäuseabschnitt 40 angeordnet ist, zwischen den magnetischen Bauteilen 23, 24 des antriebsseitigen Kopplungselements 20 angeordnet. Die Linearbewegung des antriebsseitigen Kopplungselements 20 wird aufgrund der Wechselwirkung der magnetischen Bauteile in die Linearbewegung des abtriebsseitigen Kopplungselements 21 umgesetzt, was mithilfe kontaktloser Kraftübertragung erfolgt.

Fig. 6 zeigt ein Beispiel vergleichbar der Ausführung in Fig. 5, wobei dem antriebsseitigen Kopplungselement 20 abtriebsseitig zugeordnete und im gezeigten Beispiel magnetflussführende Teile des abtriebsseitigen Kopplungselements 21 aus weichmagnetischem Material bestehen. Durch deren Materialeigenschaft, Gestaltung und / oder Abstand zum antriebsseitigen Kopplungselement 20 kann ein Übertragungsverhalten zwischen dem Antriebsmodul 4 und dem Stechwerkzeug 7a konstruktiv und durch geeignete Zusatzeinrichtungen (nicht dargestellt) beeinflusst und bedarfsgerecht eingestellt werden.

Bei den verschiedenen Beispielen sind das Antriebsmodul-Gehäuse 1a und das Nadelmodul-Gehäuse 1b, zumindest in einander gegenüberliegenden Gehäuseabschnitten 50, 51, jeweils mit einer geschlossenen Wandung (geschlossenes Gehäuse) ausgebildet. Es kann vorgesehen sein, dass zumindest das Antriebsmodul-Gehäuse 1a als ein vollständig geschlossenes Gehäuse ausgebildet ist, wobei gedichtete Kabeldurchführungen am Gehäuse 1 vorgesehen sein können. Die Gehäuseabdichtung gegenüber der Umgebung kann flüssigkeitsdicht ausgebildet sein. Auch eine Dampfdichtigkeit kann vorgesehen sein.

Es kann vorgesehen sein, dass bei geeigneter Magnetisierung, beispielsweise diametral, das antriebsseitige Kopplungselement 20 als zylindrisches Bauteil und das abtriebsseitige Kopplungselement 21 als ringförmiges Bauteil oder vice versa ausgestaltet sind.

Zudem kann das antriebsseitige Kopplungsbauteil 20 mit einer Kombination aus linearer und rotatorischer Bewegung beaufschlagt sein, um besondere Eigenschaften des Stechgeräts im Betrieb auf Haut zu erhalten. Auch hierfür kann eine vorteilhafte Magnetisierung eines oder beider Partner vorgesehen sein, beispielsweise eine segmentale und / oder eine axialdiametrale.

Bei dem Gehäuse 1 kann in einer Ausgestaltung eine mitbewegte Wandung vorgesehen sein, wobei hierzu ein Gehäusewandabschnitt aus einem flexiblen Material sein kann.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Antriebsmodul (2) für ein Handgerät zum wiederholten lokalen Aufstechen einer menschlichen oder einer tierischen Haut, mit:
- einem Antriebsmodul-Gehäuse (1a);
- einer maschinellen Antriebseinrichtung (4), die in dem Antriebsmodul-Gehäuse (1a) angeordnet und eingerichtet ist, eine Antriebskraft bereitzustellen; und
- einem antriebsseitigen Kopplungselement (20), welches in dem Antriebsmodul-Gehäuse (1a) angeordnet ist, an die Antriebseinrichtung (4) koppelt und eingerichtet ist, die bereitgestellte Antriebskraft auf ein abtriebsseitiges Kopplungselement (21), welches außerhalb des Antriebsmodul-Gehäuses (1a) an einem Nadelmodul (3) und dort an eine Stecheinrichtung (7) koppelnd angeordnet ist, kontaktlos zu übertragen.

2. Antriebsmodul nach Anspruch 1, **dadurch gekennzeichnet, dass** das antriebsseitige Kopplungselement (20) eingerichtet ist, die bereitgestellte Antriebskraft durch eine Wandung des Antriebsmodul-Gehäuses (1a) hindurch kontaktlos zu übertragen.

3. Antriebsmodul nach Anspruch 1 oder 2, **dadurch gekennzeichnet, dass** das antriebsseitige Kopplungselement (20) zum kontaktlosen Übertragen der Antriebskraft bewegbar in dem Antriebsmodul-Gehäuse (1a) gelagert ist.

4. Antriebsmodul nach mindestens einem der vorangehenden Ansprüche, **dadurch gekennzeichnet, dass** das Antriebsmodul-Gehäuse (1a) zumindest in einem vorderen Gehäuseabschnitt, in welchem das Antriebsmodul-Gehäuse (1a) an das Nadelmodul (3) koppelbar ist, ein geschlossenes Gehäuse ist.

5. Antriebsmodul nach mindestens einem der vorangehenden Ansprüche, **dadurch gekennzeichnet, dass** das antriebsseitige Kopplungselement (20) ein magnetisches Bauteil aufweist, welches eingerichtet ist, zum kontaktlosen Übertragen der Antriebskraft ein Magnetfeld bereitzustellen.

6. Antriebsmodul nach mindestens einem der vorangehenden Ansprüche, **dadurch gekennzeichnet, dass** das antriebsseitige Kopplungselement (20) einer antriebsmoduiseitigen Aufnahme (41), in welcher das abtriebsseitige Kopplungselement (21) bei angekoppeltem Nadelmodul (3) wenigstens teilweise angeordnet ist, mehrseitig gegenüberliegend ausgebildet ist.

7. Nadelmodul (3) für ein Handgerät zum wiederholten lokalen Aufstechen einer menschlichen oder einer tierischen Haut, mit:
- einem Nadelmodul-Gehäuse (1b);
- einer Stecheinrichtung (7), die in dem Nadelmodul-Gehäuse (1b) angeordnet ist, derart, dass ein Stechwerkzeug (7a) der Stecheinrichtung (7) durch eine vorderseitige Gehäuseöffnung (9) vor und zurück bewegt werden kann; und
- einem abtriebsseitigen Kopplungselement (21), welches in dem Nadelmodul-Gehäuse (1b) angeordnet ist, an die Stecheinrichtung (7) koppelt und eingerichtet ist, zum Vor- und Zurückbewegen des Stechwerkzeugs (7a) eine maschinell erzeugte Antriebskraft mittels kontaktloser Kraftübertragung von einem antriebsseitigen Kopplungselement (20) zu empfangen, das in einem Antriebsmodul (2) und dort an eine maschinelle Antriebseinrichtung (4) koppelnd angeordnet ist.

8. Nadelmodul nach Anspruch 7, **dadurch gekennzeichnet, dass** das Nadelmodul (3) ein Einwegmodul ist.

9. Nadelmodul nach Anspruch 7 oder 8, **dadurch gekennzeichnet, dass** in dem Nadelmodul-Gehäuse (1b) eine Rückstelleinrichtung (28) angeordnet ist, welche an die Stecheinrichtung (7) koppelt und eingerichtet ist, ein Zurückverlagern des Stechwerkzeugs (7a) zwischen einer Einstechstellung und einer Ausgangstellung wenigstens zu unterstützen.

10. Nadelmodul nach mindestens einem der Ansprüche 7 bis 9, **dadurch gekennzeichnet, dass** das abtriebsseitige Kopplungselement (21) ein magnetisches Bauteil aufweist, welches eingerichtet ist, zum kontaktlosen Übertragen der Antriebskraft an ein Magnetfeld des antriebsseitigen Kopplungselementes (20) zu koppeln.

11. Nadelmodul nach mindestens einem der Ansprüche 7 bis 10, **dadurch gekennzeichnet, dass** das abtriebsseitige Kopplungselement (21) einer nadelmodulseitigen Aufnahme (31), in welcher das antriebsseitige Kopplungselement (20) bei angekoppeltem Antriebsmodul (2) wenigstens teilweise angeordnet ist, mehrseitig gegenüberliegend ausgebildet ist.

12. Handgerät zum wiederholten lokalen Aufstechen einer menschlichen oder einer tierischen Haut, mit einem Antriebsmodul (2) nach mindestens einem der Ansprüche 1 bis 6 und einem hieran lösbar gekoppelten Nadelmodul (3) nach mindestens einem der Ansprüche 7 bis 11.
